# EUROPÄISCHE PATENTSCHRIFT

(11) **EP 3 487 980 B1**
(45) Veröffentlichungstag und Bekanntmachung des Hinweises auf die Patenterteilung: **22.04.2020**
(21) Anmeldenummer: 17751624.2
(22) Anmeldetag: 14.07.2017
(51) Int. Cl.: C12M 1/22, C12M 3/00, C12M 1/00, G01N 35/10

(54) **GEBINDE**
PACK
PACK

(30) Priorität: 20.07.2016 DE 202016004498 U
(43) Veröffentlichungstag der Anmeldung: 29.05.2019
(73) Patentinhaber: Xebios Diagnostics GmbH, 40597 Düsseldorf (DE)
(72) Erfinder: DECKER, Ludwig, 41516 Grevenbroich (DE); MENTZEL, Christoph, 40227 Düsseldorf (DE)
(74) Vertreter: Kluin, Jörg-Eden
(86) Internationale Anmeldenummer: PCT/EP2017/067907
(87) Internationale Veröffentlichungsnummer: WO 2018/015310

(56) Entgegenhaltungen:
- EP-A1- 1 953 219
- EP-A1- 2 112 088
- EP-A1- 2 803 590
- EP-A2- 0 087 031
- EP-A2- 0 447 893
- DE-A1- 3 218 532
- DE-C1- 19 502 520
- US-A1- 2010 035 338
- US-A1- 2012 261 277
- DATABASE WPI Week 201141 Thomson Scientific, London, GB; AN 2011-G47887 XP002775213, & CN 201 817 471 U (MA E) 4. Mai 2011 (2011-05-04)

## Beschreibung

Die Erfindung betrifft ein Gebinde von Petrischalen.

Petrischalen sind flache, runde, durchsichtige Schalen mit übergreifenden Deckeln. Sie werden meist zur Kultivierung von Mikroorganismen und zur Zellkultur in der Biologie oder der Medizin genutzt. Zu diesem Zweck wird eine meist flache Schicht aus einem meist gelförmigen Nährmedium in der Petrischale erzeugt. Hierzu wird das Nährmedium gewöhnlich auf Agar-Basis hergestellt, in einem Autoklaven durch Erhitzen sterilisiert und noch warm und damit flüssig in die jeweilige Petrischale gegossen. Es erstarrt bei Raumtemperatur und bildet eine so genannte Agarplatte.

Beispielsweise beim klinischen oder industriellen Einsatz werden häufig eine Vielzahl von mit Nährmedium versehene Petrischalen benötigt, um beispielsweise in Versuchsreihen Mikroorganismen qualifizieren und quantifizieren zu können. Hierzu werden mit so genannten "Fertignährmedien" versehene Petrischalen in Zehn-Stück Gebinden in Schlauchbeuteln oder in Strumpffolien bereitgestellt. Zur Nutzung der einzelnen Petrischalen müssen die Schlauchbeutel oder die Strumpffolie von dem Verwender manuell entfernt werden. Insbesondere bei einem größeren Bedarf an Petrischalen ist hiermit ein nicht zu vernachlässigender Zeitbedarf verbunden.

EP 1 953 219 offenbart ein Gebinde für die Lagerung und den Transport von Petrischalen als Stapel in steriler Umhüllung. Die Umhüllung besteht aus einem Plastikfilm aus zwei Teilen, die miteinander verschweißt werden, jedoch durch Aufreißen der Beutelhälften leicht und zeitsparend geöffnet werden können. Die zwei Kunststoff-Film-Teile werden als öffenbare Umverpackung mit lösbarem Verbindungsmittel als umlaufende Schweißnaht verstanden.

Der Erfindung liegt die Aufgabe zugrunde, hier Abhilfe und ein Gebinde zu schaffen, mit welchem der durch die Öffnungsvorgänge bedingte Zeitbedarf reduziert und damit die Produktivität beispielsweise eines Labors erhöht wird.

Diese Aufgabe wird durch das in Anspruch 1 wiedergegebene Gebinde gelöst.

Das erfindungsgemäße Gebinde umfasst mindestens einen sich in einer Längsrichtung erstreckenden Stapel von Petrischalen sowie ein den Stapel zusammenhaltendes und zur Entnahme von Petrischalen aus dem Stapel vorzugsweise im Wege des Durchtrennens lösbares Verbindungsmittel. Dieses Verbindungsmittel hat im Wesentlichen die Aufgabe, den Stapel von Petrischalen gegen ein unerwünschtes Auseinanderfallen zusammenzuhalten, nicht aber die Funktion, den Stapel gegen äußere Einflüsse abzuschirmen, insbesondere steril zu halten.

Letzterem dient eine den mindestens einen Stapel von Petrischalen umgebende, zur Entnahme des mindestens einen Stapels öffenbare Umverpackung.

Die Verteilung der beiden verschiedenen Funktionalitäten "den Stapel der Petrischalen zusammenhalten" und "den Stapel der Petrischalen gegen äußere Einflüsse abschirmen, insbesondere steril halten" auf zwei verschiedene Maßnahmen, nämlich auf das lösbare Verbindungsmittel einerseits und die öffenbare Umverpackung andererseits, hat zur Folge, dass ein in der öffenbaren Umverpackung steril bereitgestellter Stapel von Petrischalen kurz vor einer weiteren Verwendung entnommen werden kann, wobei jedoch die einzelnen Petrischalen weiterhin zum Stapel zusammengehalten werden. Der so zusammengehaltene Stapel kann beispielsweise einem Magazin eines Geräts zur Laborautomation zugeführt werden, ohne dass hierbei die Gefahr besteht, dass der Stapel auseinander fällt oder die Petrischalen gar einzeln in das Magazin eingebracht werden müssen. Erst im Magazin kann im Bedarfsfalle das Verbindungsmittel gelöst werden, was sich insbesondere dann als einfach und schnell durchführbar gestaltet, wenn das Lösen - wie bevorzugt - im Wege des Durchtrennens erfolgen kann.

Es hat sich gezeigt, dass der Zeitbedarf, der zur Bestückung eines Magazins eines Geräts zur Laborautomation mit einem Stapel von Petrischalen bei solchen, die in einem erfindungsgemäßen Gebinde bereitgestellt sind, gegenüber dem eingangs zitierten Stand der Technik wesentlich reduziert ist.

Bei einer ersten Variante weist das Verbindungsmittel ein den Stapel in seiner Längsrichtung zumindest teilweise umgebendes Bandmaterial auf. Bei diesem Bandmaterial kann es sich beispielsweise um einen Streifen aus Papier, Textil, oder Folienmaterial handeln, der in Längsrichtung so um den Stapel herumgeführt ist, dass er den Boden der einen, das eine Ende des Stapels begrenzenden Petrischale und den Deckel der anderen, das andere Ende des Stapels begrenzenden Petrischale unmittelbar oder mittelbar berührt, wobei der Streifen durch Zusammenfügen seiner beiden freien Enden eine Endlosschlaufe bildet.

Das Bandmaterial kann beispielsweise in Form einer Banderole ausgebildet sein.

Bei einer zweiten Variante des erfindungsgemäßen Gebindes umfasst das Verbindungsmittel eine den Stapel in seiner Längsrichtung umschließende Schlaufenanordnung aus einem elastischen, vorzugsweise gummielastischen Material. Aufgrund der elastischen, vorzugsweise gummielastischen Materialeigenschaften kann die Schlaufenanordnung leicht um den Stapel herumgelegt werden und es ist gewährleistet, dass die einzelnen Petrischalen des Stapels unter Wirkung der elastischen Kraft aufeinander gedrückt werden. Auch ist es bei entsprechend hoher Dehnbarkeit der Schlaufenanordnung möglich, eine Schlaufenanordnung mit im entspannten Zustand bestimmten Schlaufenumfang für verschieden dimensionale Stapel, insbesondere für verschieden lange Stapel, zu verwenden.

Ein besonders zuverlässiger Zusammenhalt der einen Stapel bildenden Petrischalen wird erzielt, wenn - wie bevorzugt - die Schlaufenanordnung zwei sich kreuzende und an zwei Kreuzungsstellen miteinander verbundene Schlaufenelemente umfasst. Fallen die Kreuzungsstellen bei regelgerechter Montage der Schlaufenanordnung mit einer Längsmittelachse des Stapels etwa zusammen, so wird der Stapel auf seiner Mantelfläche von um etwa 90° voneinander beabstandeten Schlaufensträngen umgeben, was zu einem besonders guten Zusammenhalt der einzelnen Petrischalen führt, der auch nicht beispielsweise durch ein Umkippen auf einer Arbeitsfläche oder anderweitige ungeschickte Handhabung verloren geht. Ein mit einer derartigen Schlaufenanordnung zusammengehaltener Stapel von Petrischalen kann sicher in ein Magazin eines Analysegeräts oder eines Bioreaktors eingesetzt werden, ohne dass hierfür besondere Sorgfalt erforderlich wäre. Hierdurch lässt sich die für ein Bestücken eines Magazins erforderliche Zeitdauer wesentlich reduzieren.

Der Zusammenhalt der Petrischalen zum Stapel kann bei beiden Ausführungsformen durch einfaches Durchtrennen des Bandmaterials oder der Schlaufenanordnung beispielsweise im Bereich einer Kreuzungsstelle beispielsweise mit einem Skalpell aufgehoben werden.

Bei einer besonders bevorzugten Ausführungsform eines erfindungsgemäßen Gebindes umfasst die Umverpackung ein im ungeöffneten Zustand steriles Innenvolumen. Aufgrund dieser Maßnahme kann der mindestens eine von einer Umverpackung umgebene Stapel über einen längeren Zeitraum lagerfähig sein. Es ist somit möglich, in einem speziell hierfür eingerichteten Betrieb Petrischalen mit Nährmedien zu befüllen, zum Stapel zusammenzufassen und mit Umverpackungen zu versehen, um diese Stapel dann beispielsweise auf dem Speditionswege örtlich entfernten Nutzern wie Laboratorien bereitzustellen.

Die Umverpackung kann eine Folienverpackung umfassen.

Vorzugsweise ist die Umverpackung manuell aufreißbar ausgebildet, wodurch die für die Bereitstellung der Stapel von Petrischalen vom Verwender benötigte Zeit abermals reduziert ist.

Es hat sich gezeigt, dass es für die Handhabbarkeit besonders von Vorteil ist, wenn der mindestens eine Stapel zwischen 45, vorzugsweise zwischen 10 und 30, besonders bevorzugt etwa 20 Petrischalen umfasst, da einerseits diese Anzahl von Petrischalen leicht zu einem Stapel zusammenzufassen und zusammenzuhalten ist, andererseits Magazingrößen existieren, die diese Anzahlen oder Teile hiervon pro Stapel aufnehmen können. Grundsätzlich ist eine Anzahl von Petrischalen pro Stapel besonders bevorzugt, die derjenigen entspricht, die ein Magazin, für die der jeweilige Stapel vorgesehen ist, in Stapelform aufnehmen kann. Der von dem Verbindungsmittel zusammengehaltene Stapel kann dann in das Magazin eingeführt werden. Erst im Magazin kann dann das Verbindungsmittel, beispielsweise mit Hilfe eines Schneidwerkzeugs, sofern erforderlich, durchtrennt werden und die einzelnen Petrischalen beispielsweise im Analysegerät oder im Bioreaktor einzeln der weiteren Verwendung zugeführt werden.

Des Weiteren bevorzugt ist ein erfindungsgemäßes Gebinde, bei welchem die Umverpackung zwischen zwei und 20, bevorzugt sechs oder 12 Stapel von Petrischalen umfasst, da diese Anzahlen von Stapeln leicht handhabbar sind und meist in symmetrischer Weise zum Zwecke der Platzeinsparung angeordnet werden können.

Wegen der besonders guten Handhabbarkeit besonders bevorzugt ist ein Gebinde, bei welchem entweder sechs Stapel in zwei Reihen zu je drei Stapel oder 12 Stapel in vier Reihen zu je drei Stapel in der Umverpackung angeordnet sind.

Insbesondere dann, wenn erfindungsgemäße Gebinde über größere Distanzen zwischen der Bereitstellung und der Verwendung transportiert werden müssen, ist es besonders bevorzugt, eine mindestens eine Umverpackung aufnehmende und diese zumindest teilweise umgebende Kartonage vorzusehen. Um diese Kartonage wieder verwenden zu können, ist diese vorzugsweise zerstörungsfrei öffen- und wiederverschließbar ausgebildet.

Die Kartonage ist ebenfalls bevorzugt derart ausgebildet, dass sie vier Reihen zu je drei Stapel von jeweils vorzugsweise etwa 10 oder etwa 20 Petrischalen umgibt. Es kann dann entweder eine Umverpackung mit vier Reihen zu je drei Stapel oder zwei Umverpackungen mit jeweils zwei Reihen zu je drei Stapel in einer Kartonage transportiert werden.

Um die mit einer Kartonage transportierten Stapel vor Beschädigungen zu schützen, ist bei einer weiteren, bevorzugten Ausführungsform eines erfindungsgemäßen Gebindes zwischen der Unterseite der Stapel und der Kartonage und vorzugsweise auch zwischen der Oberseite der Stapel und der Kartonage jeweils eine Dämpfungslage vorgesehen, die beispielsweise eine Luftpolsterfolie umfassen kann.

Die Erfindung soll nun anhand der beigefügten Zeichnungen, in der ein Ausführungsbespiel eines erfindungsgemäßen Gebindes dargestellt ist, weiter erläutert werden. Es zeigen:

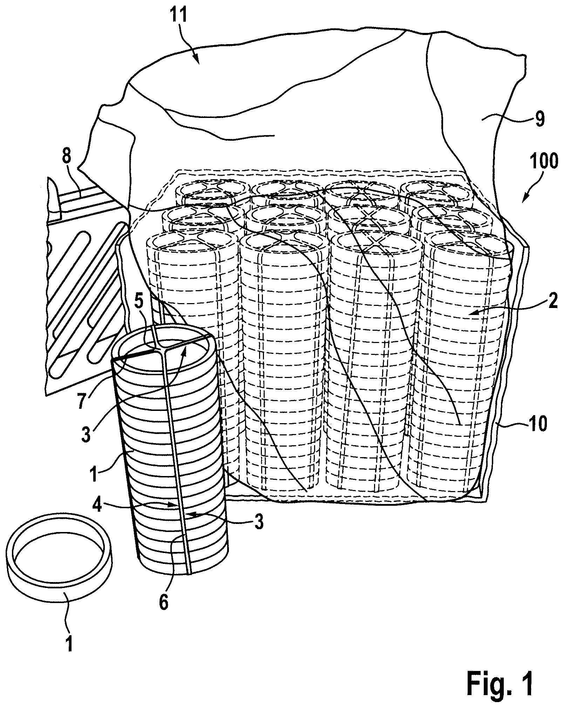

- Fig. 1: eine perspektivische Ansicht einer geöffneten Kartonage mit vier Reihen zu je drei Stapeln in einer Umverpackung befindlicher Petrischalen sowie einem in Blickrichtung davor befindlichen Stapel und einem in Blickrichtung dahinter befindlichen Magazin - teilweise von der Kartonage verdeckt -;

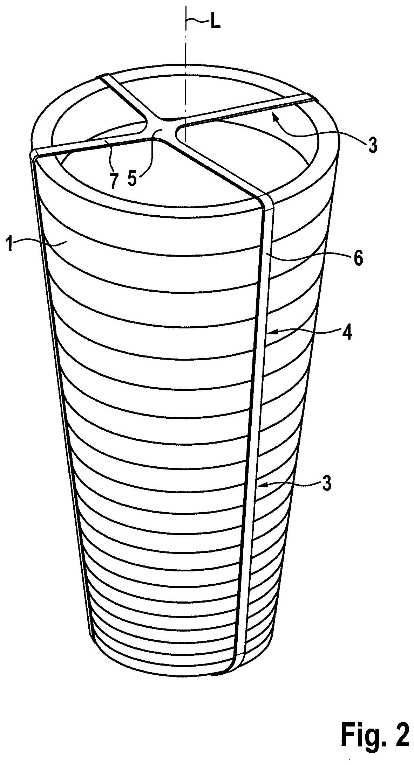

- Fig. 2: einen mit einem Ausführungsbeispiel eines Verbindungsmittels zusammengehaltenen Stapel von Petrischalen in einer perspektivischen Einzeldarstellung; sowie
- Fig. 3: einen mit einem anderen Ausführungsbeispiel eines Verbindungsmittels zusammengehaltenen Stapel von Petrischalen in einer perspektivischen Einzeldarstellung.

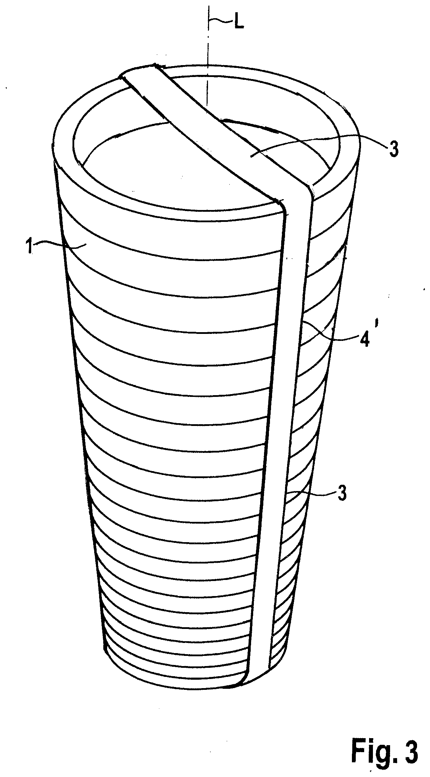

Das in der Zeichnung dargestellte und als Ganzes mit 100 bezeichnete Ausführungsbeispiel des erfindungsgemäßen Gebindes umfasst eine Mehrzahl von Stapeln 2 von Petrischalen 1, die mit in der Zeichnung nicht dargestellten Fertignährmedien befüllt sind. Jeder Stapel erstreckt sich in einer Längsrichtung L. Die zu einem Stapel 2 gehörenden Petrischalen 1 sind mit Hilfe eines lösbaren Verbindungsmittels 3 zusammengehalten. Es umfasst in einer Ausführungsform eine Schlaufenanordnung 4 aus einem gummielastischen Material, die zwei sich kreuzende und an zwei Kreuzungsstellen, von denen lediglich die obere Kreuzungsstelle 5 erkennbar ist, miteinander kreuzende Schlaufenelemente 6, 7. Anstatt durch die Schlaufenanordnung kann das Verbindungsmittel 3 auch durch ein den Stapel in seiner Längsrichtung zumindest teilweise umgebendes Bandmaterial 4' gebildet sein. Eine solche weitere Ausführungsform ist in Fig. 3 dargestellt. Das Verbindungsmittel 3 lässt sich nach Einführen des Stapels 2 in ein Magazin 8 eines Geräts zur Laborautomation, beispielsweise eines Analysegeräts leicht lösen, indem es beispielsweise mittels eines Schneidwerkzeugs, beispielsweise mittels eines Skalpells - beim Ausführungsbeispiel mit Schlaufenanordnung 4 vorzugsweise im Bereich der oberen Kreuzungsstelle 5 - durchtrennt wird und hiernach die Petrischalen einzeln aus dem Magazin 8 der Verwendung im Analysegerät zugeführt werden können.

Um die Stapel von Petrischalen von äußeren Einflüssen abschirmen, insbesondere steril halten zu können, umfasst das in der Zeichnung dargestellte Ausführungsbeispiel des erfindungsgemäßen Gebindes 100 des Weiteren eine Umverpackung 9, die aus einem Kunststofffolienmaterial gefertigt ist. Sie umgibt bei dem in der Zeichnung dargestellten Ausführungsbeispiel einen in vier mal drei Reihen angeordneten Stapel 2. Die Umverpackung 9 weist mit anderen Worten ein Innenvolumen 11 auf, in welchem sich die Stapel befinden. Die Umverpackung 9 ist bei der Bereitstellung des Gebindes gegen die äußere Umgebung verschlossen. Sie ist mit Hilfe eines Schneidwerkzeugs, beispielsweise eines Skalpells, leicht öffenbar. Ebenso kann eine in der Zeichnung nicht erkennbare Stelle vorgesehen sein, an welcher ein manuelles Aufreißen durch eine Materialschwäche, einen Einschnitt oder ähnliches erleichtert ist.

Darüber hinaus umfasst das in der Zeichnung dargestellte Ausführungsbeispiel eines erfindungsgemäßen Gebindes 100 eine Kartonage 10, die die vier mal drei Stapel 2 von Petrischalen 1 samt Umverpackung 9 aufnimmt und im geschlossenen Zustand eng umschließt. Die Kartonage ist derart ausgebildet, dass sie zerstörungsfrei öffen- und widerverschließbar ist. Zwischen den Ober- und Unterseiten der Stapel und der Kartonage können in deren geschlossenem Zustand in der Zeichnung nicht dargestellte Dämpfungslagen vorgesehen sein.

### Bezuaszeichenliste:

- 100: Ausführungsbeispiel
- 1: Petrischalen
- 2: Stapel
- 3: Verbindungsmittel
- 4: Schlaufenanordnung
- 5: obere Kreuzungsstelle
- 6: Schlaufenelement
- 7: Schlaufenelement
- 8: Magazin
- 9: Umverpackung
- 10: Kartonage
- 11: Innenvolumen
- L: Längsrichtung

## Patentansprüche

1. Gebinde (100) umfassend:
mindestens einen sich in Längsrichtung (L) erstreckenden Stapel (2) von Petrischalen (1),
ein den Stapel (2) zusammenhaltendes und zur Entnahme von Petrischalen (1) aus dem Stapel (2) vorzugsweise im Wege des Durchtrennens lösbares Verbindungsmittel (3),
und eine den mindestens einen Stapel (2) umgebende, zur Entnahme des mindestens eines Stapels (2) öffenbare Umverpackung (9),
wobei das Verbindungsmittel (3) entweder ein den Stapel (2) in seiner Längsrichtung (L) zumindest teilweise umgebendes Bandmaterial umfasst und/oder das Verbindungsmittel (3) eine den Stapel (2) in seiner Längsrichtung (L) umschließende Schlaufenanordnung (4) aus einem elastischen, vorzugsweise gummielastischen Material, umfasst.

2. Gebinde nach Anspruch 1, **dadurch gekennzeichnet, dass** das Bandmaterial eine Banderole umfasst.

3. Gebinde nach Anspruch 1 oder 2, **dadurch gekennzeichnet, dass** die Schlaufenanordnung (4) zwei sich kreuzende und an zwei Kreuzungsstellen (5) miteinander verbundene Schlaufenelemente (6, 7) umfasst.

4. Gebinde nach einem der Ansprüche 1 bis 3, **dadurch gekennzeichnet, dass** die Umverpackung (9) ein im ungeöffneten Zustand steriles Innenvolumen (11) aufweist.

5. Gebinde nach einem der Ansprüche 1 bis 4, **dadurch gekennzeichnet, dass** die Umverpackung eine Folienverpackung umfasst.

6. Gebinde nach einem der Ansprüche 1 bis 5, **dadurch gekennzeichnet, dass** die Umverpackung (9) manuell aufreißbar ausgebildet ist.

7. Gebinde nach einem der Ansprüche 1 bis 6, **dadurch gekennzeichnet, dass** der mindestens eine Stapel (2) zwischen 45, vorzugsweise zwischen 10 und 30, besonders bevorzugt 20 Petrischalen umfasst.

8. Gebinde nach einem der Ansprüche 1 bis 7, **dadurch gekennzeichnet, dass** die Umverpackung (9) zwischen zwei und 20, bevorzugt sechs oder 12 Stapel umfasst.

9. Gebinde nach Anspruch 8, **dadurch gekennzeichnet, dass** die sechs Stapel in zwei Reihen zu je drei Stapel oder die 12 Stapel in vier Reihen zu je drei Stapel in der Umverpackung angeordnet sind.

10. Gebinde nach einem der Ansprüche 1 bis 9, **gekennzeichnet durch** eine mindestens eine Umverpackung (9) aufnehmende und zumindest teilweise umgebende Kartonage (10).

11. Gebinde nach Anspruch 10, **dadurch gekennzeichnet, dass** die Kartonage (10) zerstörungsfrei öffen- und wiederverschließbar ausgebildet ist.

12. Gebinde nach Anspruch 10 oder 11, **dadurch gekennzeichnet, dass** die Kartonage (10) zur eng umschließenden Aufnahme von vier Reihen zu je drei Stapeln von jeweils vorzugsweise 10 oder 20 Petrischalen, umgeben von mindestens einer Umverpackung (9), ausgebildet ist.

13. Gebinde nach einem der Ansprüche 10 bis 12, **dadurch gekennzeichnet, dass** zwischen der Unterseiten der Stapel (2) und der Kartonage (10) und vorzugsweise zwischen der Oberseite der Stapel (2) und der Kartonage (10) jeweils eine Dämpfungslage, vorzugsweise eine Luftpolsterfolie umfassend, vorgesehen ist.

## Claims

1. Pack (100) comprising:
at least one stack (2) of Petri dishes (1) that extends in the longitudinal direction (L);
a connecting means (3) that holds the stack (2) together and is detachable, preferably by severing, in order to remove Petri dishes (1) from the stack (2);
and an outer packaging (9) that surrounds the at least one stack (2) and can be opened in order to remove the at least one stack (2),
the connecting means (3) comprising either a strip material that at least partially surrounds the stack (2) in the longitudinal direction (L) thereof and/or the connecting means (3) comprising a loop arrangement (4) that encompasses the stack (2) in the longitudinal direction (L) thereof and is made of an elastic, preferably rubber-elastic material.

2. Pack according to claim 1, **characterized in that** the strip material comprises a sleeve.

3. Pack according to either claim 1 or claim 2, **characterized in that** the loop arrangement (4) comprises two loop elements (6, 7) which intersect and are interconnected at two intersections (5).

4. Pack according to any of claims 1 to 3, **characterized in that** the outer packaging (9) has an inner volume (11) that is sterile when in the unopened state.

5. Pack according to any of claims 1 to 4, **characterized in that** the outer packaging comprises a film packaging.

6. Pack according to any of claims 1 to 5, **characterized in that** the outer packaging (9) is designed such that it can be manually torn open.

7. Pack according to any of claims 1 to 6, **characterized in that** the at least one stack (2) comprises between 45, preferably between 10 and 30, particularly preferably 20 Petri dishes.

8. Pack according to any of claims 1 to 7, **characterized in that** the outer packaging (9) comprises between two and 20, preferably six or 12 stacks.

9. Pack according to claim 8, **characterized in that**, in the outer packaging, the six stacks are arranged in two rows of three stacks each or the 12 stacks are arranged in four rows of three stacks each.

10. Pack according to any of claims 1 to 9, **characterized by** a cardboard box (10) that receives and at least partially surrounds at least one outer packaging (9).

11. Pack according to claim 10, **characterized in that** the cardboard box (10) is designed to be non-destructively openable and resealable.

12. Pack according to either claim 10 or claim 11, **characterized in that** the cardboard box (10) is designed to receive, in a tightly encompassing manner, four rows of three stacks each of preferably 10 or 20 Petri dishes, surrounded by at least one outer packaging (9).

13. Pack according to any of claims 10 to 12, **characterized in that** an absorption layer, preferably comprising an air cushion film, is provided in each case between the lower face of the stack (2) and the lower face of the cardboard box (10) and preferably between the upper face of the stack (2) and the upper face of the cardboard box (10).

## Revendications

1. Gerbe (100) comprenant :
au moins une pile (2) de boîtes de Pétri (1) qui s'étend dans la direction longitudinale (L),
un moyen de liaison (3) maintenant la pile (2) ensemble et amovible, de préférence par disjonction, pour permettre de retirer les boîtes de Pétri (1) de la pile (2), et
un suremballage (9) enveloppant l'au moins une pile (2) et pouvant s'ouvrir pour permettre de retirer ladite au moins une pile (2),
le moyen de liaison (3) comprenant soit un matériau en bande enveloppant au moins partiellement la pile (2) dans sa direction longitudinale (L) et/ou le moyen de liaison (3) comprenant un agencement de boucles (4) constitué d'un matériau élastique, de préférence en caoutchouc élastique, qui entoure la pile (2) dans sa direction longitudinale (L).

2. Gerbe selon la revendication 1, **caractérisée en ce que** le matériau en bande comprend une banderole.

3. Gerbe selon la revendication 1 ou 2, **caractérisée en ce que** l'agencement de boucles (4) comprend deux éléments à boucles (6, 7) qui se croisent et sont reliés au niveau de deux points de croisement (5).

4. Gerbe selon l'une des revendications 1 à 3, **caractérisée en ce que** le suremballage (9) présente un volume intérieur (11) stérile à l'état non ouvert.

5. Gerbe selon l'une des revendications 1 à 4, **caractérisée en ce que** le suremballage comprend un film d'emballage.

6. Gerbe selon l'une des revendications 1 à 5, **caractérisée en ce que** le suremballage (9) est conçu pour être déchiré manuellement.

7. Gerbe selon l'une des revendications 1 à 6, **caractérisée en ce que** l'au moins une pile (2) comprend 45, de préférence entre 10 et 30, de manière particulièrement préférée 20 boîtes de Pétri.

8. Gerbe selon l'une des revendications 1 à 7, **caractérisée en ce que** le suremballage (9) comprend entre deux et 20, de préférence six ou 12 piles.

9. Gerbe selon la revendication 8, **caractérisée en ce que** les six piles sont disposées en deux rangées de trois piles, ou **en ce que** les 12 piles sont disposées en quatre rangées de trois piles dans le suremballage.

10. Gerbe selon l'une des revendications 1 à 9, **caractérisée par** au moins un suremballage (9) recevant et enveloppant au moins partiellement la boîte en carton (10).

11. Gerbe selon la revendication 10, **caractérisée en ce que** la boîte en carton (10) est conçue pour pouvoir être ouverte et refermée de manière non destructive.

12. Gerbe selon la revendication 10 ou 11, **caractérisée en ce que** la boîte en carton (10) est conçue pour enfermer étroitement quatre rangées de trois piles de 10 ou 20 boîtes de Pétri chacune de préférence, enveloppées par au moins un suremballage (9).

13. Gerbe selon l'une des revendications 10 à 12, **caractérisée en ce que**, entre les côtés inférieurs des piles (2) et la boîte en carton (10) et de préférence entre le côté supérieur des piles (2) et la boîte en carton (10), une couche d'amortissement, comprenant de préférence un papier à bulles, est prévue dans chaque cas.
